## Europäisches Patentamt
## European Patent Office
## Office européen des brevets

(19)

(11) Veröffentlichungsnummer: **0 161 586**
**B1**

(12) **EUROPÄISCHE PATENTSCHRIFT**

(45) Veröffentlichungstag der Patentschrift:
02.11.88

(51) Int. Cl.⁴: **G 01 N 33/00**

(21) Anmeldenummer: **85105354.6**

(22) Anmeldetag: **02.05.85**

(54) Einrichtung zur Durchführung von Kalibrierungen an Fernmessköpfen für Gasmessgeräte.

(30) Priorität: **12.05.84 DE 3417769**

(43) Veröffentlichungstag der Anmeldung:
**21.11.85 Patentblatt 85/47**

(45) Bekanntmachung des Hinweises auf die Patenterteilung:
**02.11.88 Patentblatt 88/44**

(84) Benannte Vertragsstaaten:
**CH FR GB LI**

(56) Entgegenhaltungen:
**DE-A-3 126 647**
**US-A-4 151 738**

**Die Akte enthält technische Angaben, die nach dem Eingang der Anmeldung eingereicht wurden und die nicht in dieser Patentschrift enthalten sind.**

(73) Patentinhaber: **AUERGESELLSCHAFT GMBH, Thiemannstrasse 1, D-1000 Berlin 44 (DE)**

(72) Erfinder: **Kloft, Eva- Maria, Braukweg 26, D-4370 Marl- Polsum (DE)**
Erfinder: **Albrecht, Peter, Hochwaldsteig 10, D-1000 Berlin 22 (DE)**
Erfinder: **Breitenfeld, Günter, Endestrasse 24, D-1000 Berlin 39 (DE)**

## Beschreibung

Die vorliegende Erfindung betrifft eine Einrichtung zur Kalibrierung von Gasmeß- und Gaswarnanlagen nach dem Oberbegriff des Anspruchs 1.

Es ist bekannt, daß Gaswarnanlagen in gewissen Abständen kalibriert werden müssen. Hierfür werden Prüfgase bekannter Konzentrationen verwendet, die von einer Bedienungsperson manuell direkt am zu kalibrierenden Fernmeßkopf aufgegeben werden müssen. Das Kalibrieren erfordert regelmäßig mindestens zwei Bedienungspersonen. Eine Person, die die Fernmeßköpfe abschreitet und das Prüfgas z. B. aus einer Druckgasflasche am Meßkopf aufgibt, und eine andere Person in der Zentrale, die die Kalibrierung (z. B. Einstellung von Nullpunkt und Empfindlichkeit) am Gasmeßmeßgerät vornimmt. Die Kalibrierung ist besonders aufwendig, wenn die Fernmeßköpfe und die dazugehörigen Anzeige- und Registriergeräte anlagenbedingt räumlich weit voneinander getrennt angeordnet sind.

Weiterhin ist aus der DE-A-3 126 647 bekannt, daß zur zentralen Überwachung und Kalibrierung außenliegender Sensoren von der zentralen Stelle aus jeweils Zuführungsleitungen zur Zuführung von Nullpunktgas bzw. Kalibriergas an die außenliegenden Sensorköpfe angeschlossen sind. Die elektrischen Fernmeßkabel sind von den Zuführungsleitungen getrennt an die außenliegenden Sensorköpfe geführt und an diese angeschlossen.

Der Erfindung liegt daher die Aufgabe zugrunde, eine Einrichtung zur Kalibrierung von Gasmeß- und Gaswarnanlagen der vorhin genannten Art zu verbessern.

Diese Aufgabe wird gemäß der Erfindung durch die Merkmale des Anspruchs 1 gelöst.

Weitere vorteilhafte Ausgestaltungen der Erfindung ergeben sich aus den abhängigen Ansprüchen.

Ausführungsbeispiele der Erfindung sind in der Zeichnung dargestellt und werden im folgenden näher beschrieben. Es zeigen:

Fig. 1 eine schematische Darstellung der erfindungsgemäßen Einrichtung,

Fig. 2 eine Schnittdarstellung durch die erfindungsgemäße Prüfgasleitung,

Fig. 3 eine Schnittdarstellung der Anschlußverbindung der Prüfgasleitung an einem Fernmeßkopf,

Fig. 4 eine Schnittdarstellung der Anschlußverbindung der Prüfgasleitung mit einem anderen Fernmeßkopf,

Fig. 5 eine Schnittdarstellung der Anschlußverbindung der Prüfgasleitung über einen Anschlußkasten an einen Fernmeßkopf, unter Benutzung eines aufgesetzten Prüfvorsatzes.

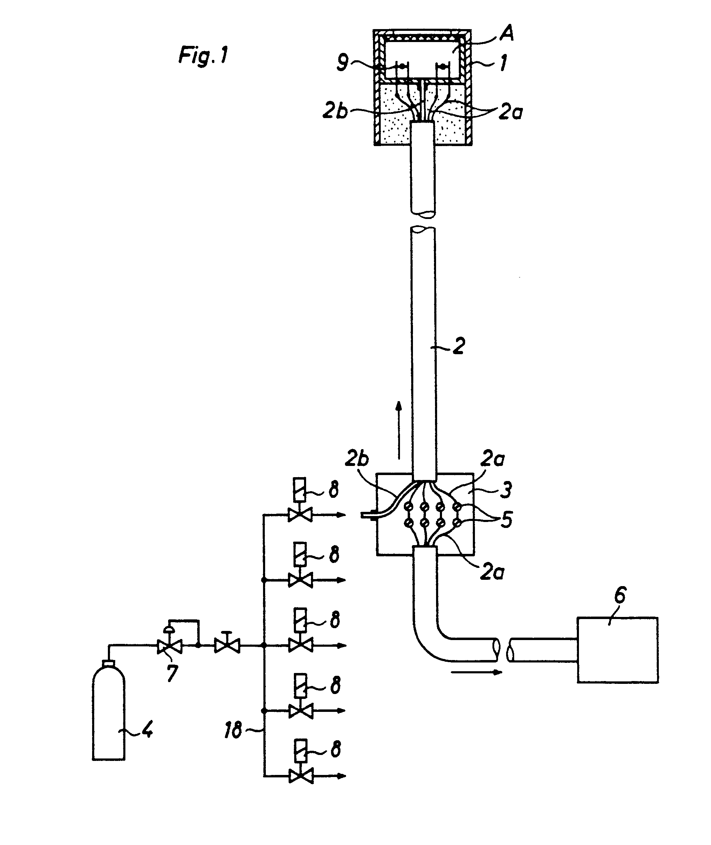

In der Fig. 1 ist stark vereinfacht das Schema einer erfindungsgemäßen zentralen Prüfgasstelle mit Prüfgaseinspleißung in die erfindungsgemäße Prüfgasleitung zu den räumlich weit voneinander getrennt angeordneten Fernmeßköpfen dargestellt.

Mit 1 ist einer der Fernmeßköpfe bezeichnet und mit 2 ein Fernmeßkabel, in dem zusätzlich zu den elektrischen Leitungen 2a eine oder mehrere Prüfgasleitungen 2b angeordnet sind. Das Fernmeßkabel 2 endet in der zentralen Prüfgasstelle in einem Verbindungskasten 3 mit einer Prüfgaseinspleißung, zum Anschluß an eine Druckgasflasche 4 mit Prüfgas oder an eine andere Prüfgasquelle, und mit Anschlußklemmen 5, zum Anschluß der elektrischen Leitungen 2a an ein Gasmeßgerät 6. Die Prüfgaseinspleißung erfolgt im Falle der Druckgasflasche 4 über Verbindungsleitungen 18 mit dazwischengeschaltetem Druckminderer 7 und Prüfgasumschalter 8 zum Verbindungskasten 3.

Die Prüfgasumschalter 8 sind beispielsweise manuell oder automatisch zu betätigende Magnetventile. Die automatische Steuerung kann über Rechner oder Zeitschalter erfolgen.

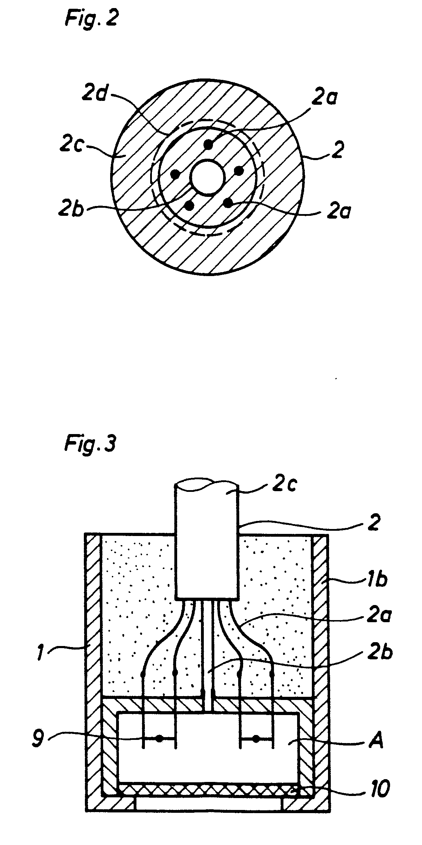

Die Figur 2 zeigt einen Querschnitt durch das Fernmeßkabel 2. Mit 2a sind die elektrischen Leitungen und mit 2b ist die Prüfgasleitung bezeichnet. Die elektrischen Leitungen 2a können beispielsweise konzentrisch um die Prüfgasleitung 2b angeordnet sein. Das Kabel 2 weist ferner eine Isolation 2c und einen Schirm 2d auf.

Die Figur 3 zeigt eine Anschlußverbindung des Fernmeßkabels 2 mit dem Fernmeßkopf 1. Die elektrischen Leitungen 2a und die Prüfgasleitung 2b sind zum Anschluß an die die Gasspürelemente 9 aufnehmende Meßkammer A aus der das Fernmeßkabel 2 umgebenden Isolation 2c vor der Meßkammer herausgeführt, und im Gehäuse 1b des Fernmeßkopfes vergossen. Die Meßkammer A ist mit einer gasdurchlässigen Scheibe 10 abgedeckt. Das Prüfgas wird der Meßkammer A durch die Prüfgasleitung 2b an der der gasdurchlässigen Scheibe 10 gegenüberliegenden Seite zugeführt.

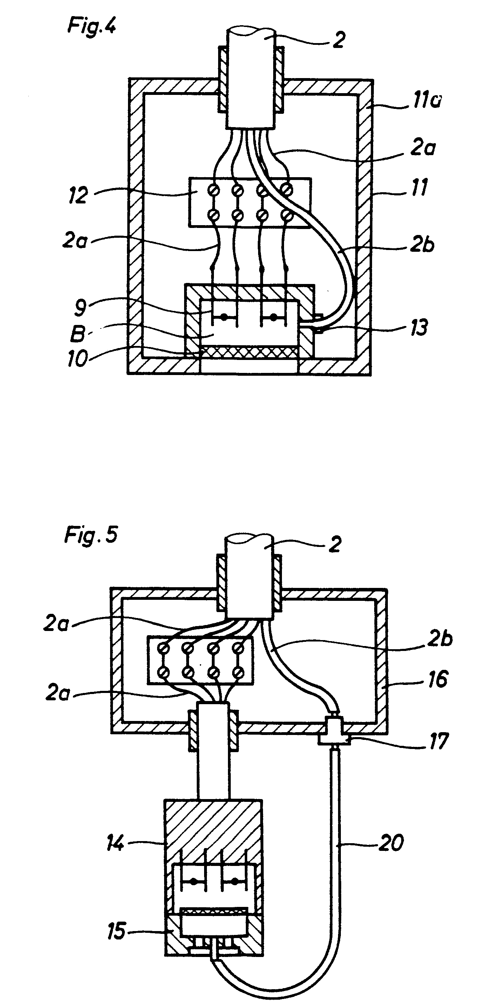

Die Figur 4 zeigt einen weiteren Fernmeßkopf 11 mit einer Anschlußverbindung des Fernmeßkabels 2 an eine Meßkammer B. Der Fernmeßkopf 11 besteht im wesentlichen aus einem Anschlußkasten 11a, in dem die Meßkammer B und einer Anschlußleiste 12 zum Anschluß der elektrischen Leitungen 2a an die Gasspürelemente 9 angeordnet sind. Die Meßkammer B ist mit einer Sintermetallscheibe 10 abgedeckt. Die Prüfgasleitung 2b ist seitlich an die Meßkammer B mittels eines Gaseinlaßnippels 13 angeschlossen.

Die Figur 5 zeigt schließlich einen Fernmeßkopf 14 mit einem Prüfvorsatz 15. Das Fernmeßkabel 2 mit den elektrischen Leitungen 2a und der Prüfgasleitung 2b enden, von der zentralen Prüfgasstelle kommend, in einem Klemmenanschlußkasten 16, an dem auch der Fernmeßkopf 14 angeschlossen ist. Die Prüfgasleitung 2b wird an eine am Klemmenanschlußkasten 16 angeordnete Schottverschraubung 17 angeschlossen, während eine weitere Prüfgasleitung 20 vom Anschlußkasten 16 an dem Prüfvorsatz 15 angeschlossen ist. Bei dieser Ausführung wird das Prüfgas von oben über den Prüfvorsatz 15 durch die gasdurchlässige Scheibe 10 des Fernmeßkopfes 14 auf die in der Meßkammer angeordneten Gasspürelemente aufgegeben.

**Patentansprüche**

1. Einrichtung zur Kalibrierung von Gasmeß- und Gaswarnanlagen (6), bei der Fernmeßköpfe (1) mit Prüfgasen bekannter Konzentrationen beaufschlagt werden, und die Fernmeßköpfe jeweils über mehradrige elektrische Fernmeßkabel (2) und Prüfgasleitungen (2b) mit der in einer Zentrale angeordneten Gasmeß- und Gaswarnanlage (6) verbunden und von dort elektrisch versorgt werden, dadurch gekennzeichnet sind, daß
a) in dem mehradrigen elektrischen Fernmeßkabel (2) konzentrisch zu den elektrischen Leitungen (2a) die Prüfgasleitung (2b) angeordnet ist, die zur Zuführung des Prüfgases an den Fernmeßkopf (1) von der zentralen Prüfgasstelle (4) aus dient, und
b) ein Verbindungskasten (3) mit Anschlußklemmen (5) zwischen der Gasmeß- und Gaswarnanlage (6) und der zentralen Prüfgasstelle (4) vorgesehen ist, in dem einerseits das von dem Fernmeßkopf (1) kommende Fernmeßkabel (2) mit den elektrischen Leitungen (2a) und dem Prüfgaskabel (2b) angeschlossen ist, und in dem andererseits die elektrische Verbindung der Gasmeß- und Gaswarnanlage (6) mit dem Fernmeßkabel sowie die Einspeisung des Prüfgases von der zentralen Prüfgasstelle aus in die Prüfgasleitung (2b) des Fernmeßkabels (2) erfolgt.
2. Einrichtung nach Anspruch 1, dadurch gekennzeichnet, daß die jeweils aus dem Verbindungskasten (3) herausgeführten Prüfgasleitungen (2b) zur Prüfgaseinspleißung an Prüfgasumschalter (8) angeschlossen sind, die über eine Verbindungsleitung (18) mit der Prüfgasquelle (4) verbunden sind.
3. Einrichtung nach Anspruch 1 und 2, dadurch gekennzeichnet, daß die Prüfgasquelle eine Druckgasflasche (4) ist, und daß die von dem Verbindungskasten (3) kommende Verbindungsleitung (18) über einen Druckminderer (7) mit der Druckgasflasche (4) verbunden ist.
4. Einrichtung nach Anspruch 1; dadurch gekennzeichnet, daß das Fernmeßkabel (2) ein Flachbandkabel ist und die elektrischen Leitungen (2a) jeweils seitlich neben der Prüfgasleitung (2b) angeordnet sind.
5. Einrichtung nach Anspruch 1, dadurch gekennzeichnet, daß die Meßkammer (A) des Fernmeßkopfes (1) mit einer gasdurchlässigen Scheibe (10) abgedeckt ist, und daß das Prüfgas durch die Prüfgasleitung (2b) der Meßkammer (A) an der der Scheibe (10) gegenüberliegenden Seite zugeführt wird.
6. Fernmeßkopf zur Durchführung der Kalibrierung nach Anspruch 1, dadurch gekennzeichnet, daß der mit der gasdurchlässigen Scheibe (10) abgedeckte Fernmeßkopf (11) aus einem Anschlußkasten (11a) mit Anschlußleiste (12) und aus der die Gasspürelemente (9) aufnehmenden Meßkammer (B) besteht, und daß die Prüfgasleitung (2b) seitlich an die Meßkammer (B) mittels eines Gaseinlaßnippels (13) angeschlossen ist.
7. Fernmeßkopf zur Durchführung der Kalibrierung nach Anspruch 1, dadurch gekennzeichnet, daß der Fernmeßkopf (14) einen Prüfvorsatz (15) aufweist und an einem Klemmen-Anschlußkasten (16) angeschlossen ist, in dem das Fernmeßkabel (2) mit den elektrischen Leitungen (2a) und der Prüfgasleitung (2b) angeschlossen ist.
8. Fernmeßkopf nach Anspruch 7, dadurch gekennzeichnet, daß am Prüfvorsatz (15) des Fernmeßkopfes (14) ein Prüfgaskabel (19) am Klemmen-Anschlußkasten (14) mittels Schottverschraubung (17) angeschlossen ist, und daß das Prüfgas über den Prüfvorsatz (15) auf die in der Meßkammer des Fernmeßkopfes angeordneten Gasspürelemente (9) aufgegeben wird.

**Claims**

1. Apparatus for calibrating gas measuring and gas warning installations (6), in which telemetering heads (1) are acted upon with test gases of known concentrations, and the telemetering heads are in each case connected by way of multi-core electrical telemetering cables (2) and test gas conduits (2b) to the gas measuring and gas warning installation (6) arranged in a power unit and are supplied electrically from there, characterised in that:
a) arranged in the multi-core electrical telemetering cable (2) concentrically with the electrical leads (2a) is the test gas conduit (2b) which serves to supply the test gas to the telemetering head (1) from the central test gas location (4), and
b) a junction box (3) with connecting terminals (5) is provided between the gas measuring and gas warning installation (6) and the central test gas location (4), in which box on the one hand the telemetering cable (2) coming from the telemetering head (1) is connected to the electrical leads (2a) and the test gas cable (2b), and in which on the other hand the electrical connection of the gas measuring and gas warning installation (6) to the telemetering cable is effected as well as the feeding in of the test gas from the central test gas point into the test gas pipe (2b) of the telemetering cable (2).
2. Apparatus according to claim 1, characterised in that the test gas pipes (2b), respectively led out of the junction box (3) for the test gas input connection are connected to test gas change-over switches (8) which are connected by way of a connecting conduit (18) to the test gas source.

3. Apparatus according to claim 1 and 2, characterised in that the test gas source is a compressed gas bottle (4), and in that the connecting conduit (18) coming from the junction box (3) is connected by way of a pressure reducer (7) to the pressure gas bottle (4).

4. Apparatus according to claim 1, characterised in that the telemetering cable (2) is a ribbon cable and the electrical leads (2a) are each arranged laterally next to the test gas conduit (2b).

5. Apparatus according to claim 1, characterised in that the measuring chamber (A) of the telemetering head (10) is covered with a gas-permeable disc (10) and in that the test gas is supplied through the test gas conduit (2b) to the measuring chamber (A) on the side lying opposite to the disc (10).

6. Telemetering head for carrying out the calibration according to claim 1, characterised in that the telemetering head (11) covered with the gas-permeable disc (10) consists of a junction box (11a) with terminal strip (12) and of the measuring chamber (B) receiving the gas detection elements (9), and in that the test gas pipe (2b) is connected laterally to the measuring chamber (B) by means of a gas inlet nipple (13).

7. Telemetering head for carrying out the calibration according to claim 1, characterized in that the telemetering head (14) has a testing mask (15) and is connected to a terminal connecting box (16) in which the telemetering cable (2) is connected to the electrical leads (2a) and the test gas conduit (2b).

8. Telemetering head according to claim 7, characterised in that on the testing mask (15) of the telemetering head (14) a test gas cable (19) is connected to the terminal connecting box (14) by means of a screw bushing (17) and in that the test gas is delivered by way of the testing mask (15) to the gas detection elements arranged in the measuring chamber of the telemetering head.

**Revendications**

1. Dispositif d'étalonnage d'installations de détection et de mesure de gaz (6), dans lequel des têtes de télémesure (1) sont sollicitées par des gaz de contrôle de concentrations connus, et les têtes de télémesure sont respectivement reliées, par l'intermédiaire d'un câble électrique de télémesure à plusieurs brins (2) et de conduits de gaz de contrôle (2b) avec l'installation de détection et de mesure de gaz (6) installée dans une centrale de manière à être alimentées en courant électrique à partir de celle-ci, caractérisé en ce que:

a) dans le câble électrique de télémesure à plusieurs brins (2), il est prévu concentriquement aux conducteurs électriques (2a) le conduit de gaz de contrôle (2b) qui sert à alimenter la tête de télémesure (1) en gaz de contrôle à partir du poste central de gaz de contrôle (4), et

b) un boîtier de liaison (3) comportant des bornes de connexion (5) est prévu entre l'installation de détection et mesure de gaz (6) et le poste central de gaz de contrôle (4) et dans ce boîtier d'une part le câble de télémesure (2) provenant de la tête de télémesure (1) est relié aux conducteurs électriques (2a) et au conduit de gaz de contrôle (2b) et d'autre part la liaison électrique de l'installation de détection et mesure de gaz (6) est effectuée avec le câble de télémesure tandis que l'alimentation en gaz de contrôle à partir du poste central de gaz de contrôle est effectuée dans le conduit de gaz de contrôle (2b) du câble de télémesure (2).

2. Dispositif selon la revendication 1, caractérisé en ce que les conduits de gaz de contrôle (2b) partant respectivement du boîtier de liaison (3) sont reliés, en vue de l'alimentation en gaz de contrôle, à des distributeurs de gaz de contrôle (8), qui sont eux-mêmes reliés avec la source de gaz de contrôle (4) par l'intermédiaire d'un conduit de liaison (18).

3. Dispositif selon une des revendications 1 et 2, caractérisé en ce que la source de gaz de contrôle est une bouteille de gaz sous pression (4) et en ce que le conduit de liaison (18) partant du boîtier de liaison (3) est relié à la bouteille de gaz sous pression (4) par l'intermédiaire d'un réducteur de pression (7).

4. Dispositif selon la revendication 1, caractérisé en ce que le câble de télémesure (2) est un câble à bande plate et les conducteurs électriques (2a) sont respectivement disposés latéralement à côté du conduit de gaz de contrôle (2b).

5. Dispositif selon la revendication 1, caractérisé en ce que la chambre de mesure (A) de la tête de télémesure (1) est recouverte par un disque (10) perméable au gaz et en ce que le gaz de contrôle est amené par le conduit de gaz de contrôle (2b) à la chambre de mesure (A) sur le côté opposé au disque (10).

6. Tête de télémesure pour effectuer l'étalonnage conformément à la revendication 1, caractérisée en ce que la tête de télémesure (11), recouverte par le disque (10) perméable au gaz, se compose d'un boîtier de liaison (11a) comportant une barrette de liaison (12) et d'une chambre de mesure (B) recevant les éléments de détection de gaz (9) et en ce que le conduit de gaz de contrôle (2b) est relié latéralement à la chambre de mesure (B) au moyen d'une tubulure d'admission de gaz (13).

7. Tête de télémesure pour effectuer l'étalonnage conformément à la revendication 1, caractérisée en ce que la tête de télémesure (14) comporte un auxiliaire de contrôle (15) et est reliée à un boîtier de connexion à bornes (16) dans lequel le câble de télémesure (2) est relié aux conducteurs électiques (2a) et au conduit de gaz de contrôle (2b).

8. Tête de télémesure selon la revendication 7, caractérisé en ce que, dans l'auxiliaire de contrôle (15) de la tête de télémesure (14), un conduit de gaz de contrôle (19) est relié au boîtier de raccordement (14) au moyen d'un raccord (17) et en ce que le gaz de contrôle est amené par l'intermédiaire de l'auxiliaire de contrôle (15) sur les éléments de détection de gaz (9) qui sont disposés dans la chambre de mesure de la tête de télémesure.

Fig.1

**Fig. 2**

**Fig. 3**

Fig.4

Fig.5